# FASCICULE DE BREVET EUROPEEN

(11) **EP 1 519 698 B1**
(45) Date de publication et mention de la délivrance du brevet: **25.02.2015**
(21) Numéro de dépôt: 03759993.3
(22) Date de dépôt: 16.05.2003
(51) Int. Cl.: A61F 5/00

(54) **SYSTEME POUR LE TRAITEMENT DE L'OBESITE ET IMPLANT POUR UN TEL SYSTEME**
SYSTEM FÜR DIE BEHANDLUNG VON FETTSUCHT UND IMPLANTAT FÜR EIN SOLCHES SYSTEM
SYSTEM FOR TREATING OBESITY AND IMPLANT THEREFOR

(30) Priorité: 13.06.2002 FR 0207283; 11.02.2003 FR 3701619
(43) Date de publication de la demande: 06.04.2005
(73) Titulaire: ACCESSURG, 74320 Sevrier (FR)
(72) Inventeur: CANCEL, Richard, F-83130 La Garde (FR); WALLACE, Richard, F-83590 Gonfaron (FR); SASSI, Gérard, F-83200 Toulon (FR); GREPILLAT, Daniel, F-74320 Sevrier (FR)
(74) Mandataire: Novagraaf Technologies
(86) Numéro de dépôt international: PCT/FR2003/001505
(87) Numéro de publication internationale: WO 2003/105732

(56) Documents cités:
- WO-A-00/15158
- WO-A-01/47432
- FR-A- 2 797 181

## Description

La présente invention concerne un système pour le traitement de l'obésité, constitué par un implant gastrique motorisé et un équipement de commande destiné à un opérateur médical.

De tels équipements sont connus dans l'état de la technique dans leurs principes généraux.

La demande de brevet PCT WO0112108 décrit un implant gastrique commandé un moyen de transmission d'énergie assurant une transmission sans fil d'énergie d'une première forme à partir de l'extérieur du corps d'un patient. Un dispositif médical implanté peut fonctionner en réponse à une énergie d'une seconde forme différente de l'énergie de la première forme. Un moyen implanté de transformation d'énergie transforme l'énergie de la première forme, transmise sans fil par le moyen de transmission d'énergie, en énergie de la seconde forme destinée à être utilisée dans la commande et l'exploitation du dispositif médical.

La demande de brevet PCT WO0015158 décrit un autre implant gastrique de constriction, au moins en partie implantable dans le corps humain ou animal, comprenant un organe de constriction formant dans sa configuration opératoire un anneau. Cet organe de constriction comporte une bande flexible, dont les deux extrémités sont adjacentes l'une par rapport à l'autre dans la configuration opératoire, ainsi qu'un moyen d'actionnement de l'organe de constriction. Une extrémité de la bande flexible comporte un élément tractable, permettant de déplacer ladite extrémité par rapport à l'autre extrémité en générant une déformation radiale de l'organe de constriction.

La demande de brevet WO0112076A1 décrit un appareil de traitement des brûlures gastriques et des reflux gastro-oesophagiens, qui comprend un dispositif d'étranglement commandable que l'on peut implanter à un patient ou à un animal, ce dispositif étant destiné à entrer en contact avec l'estomac près du cardia ou à entrer en contact avec l'oesophage de façon à former un étranglement dans la voie alimentaire. Un organe d'émission d'énergie permettant une émission d'énergie sans fil d'une première forme en provenance de l'extérieur du patient est prévu. On peut faire fonctionner ce dispositif d'étranglement par réaction à une énergie d'une seconde forme différente de l'énergie de la première forme, délivrée de façon à faire varier l'étranglement dans la voie alimentaire. Un organe de transformation d'énergie implantable est adapté pour transformer l'énergie de la première forme émise par l'organe d'émission d'énergie en une énergie de la seconde forme.

Il apparaît que la mise en oeuvre de tels implants pose des difficultés liées à la puissance électromagnétique nécessaire au bon fonctionnement de l'implant. Il est en effet important que la puissance d'induction soit suffisante pour assurer le bon fonctionnement du moteur, mais, si la puissance est trop importante, elle provoque un échauffement qui peut devenir néfaste.

Cette difficulté est augmentée par le fait que le positionnement de l'implant varie d'un patient à l'autre, et que l'orientation relative ainsi que la distance entre les antennes de l'implant et l'équipement de commande a une incidence majeure sur l'énergie transmise. Une solution consiste à séparer la bobine d'induction de l'implant, afin de permettre un positionnement de la bobine près de la peau du patient. Cela permet certes de réduire les problèmes de transmission d'énergie, mais implique une intervention chirurgicale plus lourde.

Un but de la présente invention est d'éviter une telle intervention chirurgicale, et d'éviter au patient d'avoir sous la peau une antenne, ladite antenne devant en plus être physiquement reliée à l'implant.

Un autre but de l'invention est de remédier au problème de l'adéquation de l'énergie transmise, et à augmenter la sécurité d'utilisation des anneaux gastriques.

À cet effet, l'invention concerne, selon son acceptation la plus générale, un système pour le traitement de l'obésité comprenant un implant gastrique pour le contrôle de l'absorption alimentaire et un équipement de commande, l'implant gastrique comportant un anneau apte à enserrer l'oesophage et un moyen de commande motorisé pour l'ajustement de l'extension dudit anneau, ainsi qu'une bobine d'induction pour l'alimentation sans contact du moyen de commande motorisé caractérisé en ce que la bobine d'induction de l'implant est intégrée à l'implant et en ce que l'équipement de commande comporte des moyens pour l'ajustement de la puissance d'émission et en ce que l'implant comporte un circuit électronique délivrant un signal de réponse à une commande transmise par l'équipement de commande.

Avantageusement, l'implant comporte :
- une mémoire pour l'enregistrement de données numériques d'identification de l'implant [date de fabrication, numéro de série, ...] ;
- et/ou une mémoire pour l'enregistrement de données numériques d'identification du patient [date d'implantation, identifiant, , , ...] ;
- et/ou une mémoire pour l'enregistrement de données numériques correspondant à l'historique [date de modification, position] ;
- et/ou une mémoire pour l'enregistrement de la position absolue de l'implant ;
- et/ou une mémoire pour l'enregistrement de limites de déplacement de l'anneau, et un moyen pour empêcher la commande du moteur en cas de réception de commandes conduisant à un dépassement desdites limites.

Selon une variante préférée, l'équipement de commande comprend des moyens de réception des informations enregistrées dans la mémoire d'un implant et une mémoire pour l'enregistrement par exemple de l'historique, comprenant l'identifiant de chacun des implants commandés et la date de modification de chacun desdits implants.

Avantageusement, l'équipement de commande comprend une antenne mobile et un moyen de génération d'une trame numérique de référence pour l'étape d'ajustement de la puissance d'émission préalable à l'étape de commande d'un anneau implanté.

De préférence, l'équipement de commande comprend un circuit de modulation en amplitude de la tension d'alimentation de l'antenne mobile.

Selon une variante particulière, l'implant comporte un circuit de temporisation activant le mode veille après une période prédéterminée sans signaux de commande.

Selon une autre variante, le système comprend une fonction de test commandant le déplacement de l'implant, à savoir la fermeture puis l'ouverture d'une position.

De préférence, l'équipement de commande comporte un calculateur pour la fabrication d'un signal de commande qui est fonction d'une position saisie par un opérateur et les informations de contrôle provenant de l'implant à commander.

Selon un mode de mise en oeuvre préféré, l'équipement de commande est apte à générer un signal sécurisé pour le déplacement d'un pas du moteur de l'implant.

L'invention sera mieux comprise à la lecture de la description qui suit, se référant aux dessins annexés concernant un exemple non limitatif de réalisation, où :
- la figure 1 représente une vue en coupe d'un implant selon l'invention ;
- la figure 2 représente le schéma de principe de l'équipement de commande ;
- la figure 3 représente le schéma de principe des circuits électronique de l'implant ;
- la figure 4 représente l'algorithme de fonctionnement de l'équipement de commande ;
- la figure 5 représente l'algorithme de fonctionnement de l'implant.
- la figure 6 représente un schéma de principe d'une variante de réalisation de l'équipement de commande (console de pilotage - émetteur / récepteur).

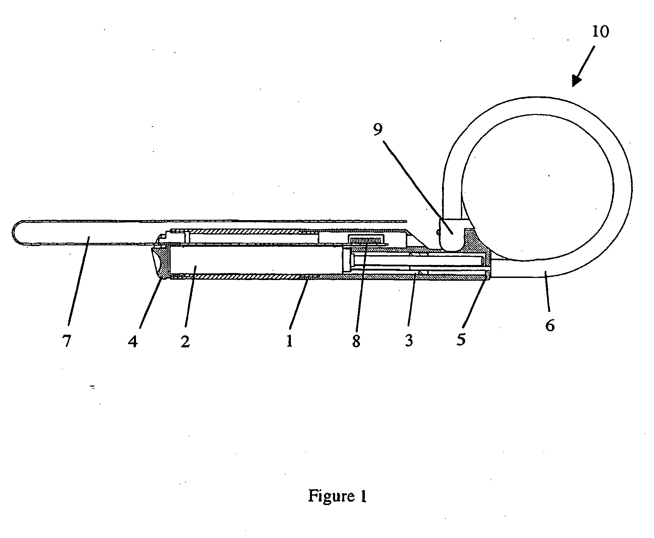

L'implant représenté en figure 1 en vue de coupe comprend un boîtier (1) d'une section d'environ 10 mm jusqu'à 20 mm pour permettre de loger l'antenne dans le boîtier et pour permettre une implantation par coelioscopie. Ce boîtier fermé par un bouchon (4) incorpore un circuit électronique (8) un moteur sans balais « brushless » (2) entraînant un manchon d'accouplement fileté (3) susceptible de se déplacer en translation dans un bec tubulaire (5). Ce manchon fileté (3) prolonge l'une des extrémités d'une bande (6) formant la boucle gastrique (10). Cette boucle présente une longueur déployée de 85 millimètres. De préférence, le périmètre maximal est de 85 millimètres. L'extrémité opposée au manchon fileté (3) présente un crochet pour l'accrochage au corps du boîtier (1) après implantation autour de l'oesophage.

L'implant comporte en outre une bobine d'induction constituée par un support de forme rectangulaire présentant des spires conductrices.

La course du manchon fileté (3) est d'environ 15 à 25 millimètres. La longueur du boîtier est d'environ 70 à 85 millimètres. La longueur de la bobine d'induction est d'environ 67 à 82 millimètres. La vitesse de déplacement est d'environ 12 à 25 mm par minutes, avec une force de 8 à 12 newtons. La course est divisée en 8 positions réparties linéairement, avec une précision d'environ 10 %. Suivant un premier mode de réalisation de l'invention, le moteur comprend un encodeur formé par exemple par une sonde de hall détectant le déplacement angulaire et délivrant un signal de déplacement relatif au circuit électronique de l'implant. Suivant un second mode de réalisation de l'invention, c'est l'électronique de pilotage qui reconstitue la position angulaire du moteur puis, par comptage, la position du manchon fileté.

Le circuit électronique intégré dans l'implant comprend une mémoire vive non volatile pour l'enregistrement, avant l'implantation, des informations non modifiables après le verrouillage de chacune d'elles, telles que :
- le numéro de série de l'implant ;
- l'identifiant du patient ;
- la date de fabrication ;
- la date de première mise en service ;
ainsi que des informations modifiables après l'implantation :
- la date de la dernière intervention ;
- l'historique des N dernières interventions, par exemple des 8 dernières interventions ;
- le nombre d'interventions depuis la mise en service.

L'implant est positionné de façon à ce que l'anneau entoure l'oesophage, et non pas l'estomac.

Son fonctionnement est commandé par un équipement de commande comprenant une console de pilotage et une antenne mobile pouvant être positionnée à proximité de ou de préférence sur l'abdomen du patient, avec une faible possibilité d'orientation angulaire.

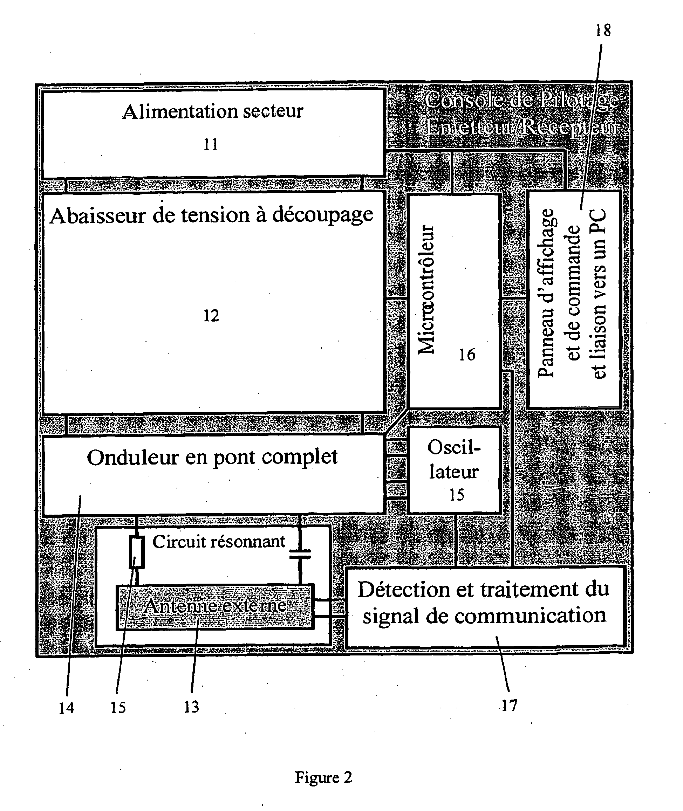

L'équipement de commande dont le schéma de principe est représenté en figure 2 comprend une alimentation à découpage (11, 12) avec un circuit abaisseur de tension à découpage (12). L'antenne (13) est couplée à un onduleur en pont complet (14) par un circuit accordé (15).

L'énergie transmise à l'antenne (13) est modulée ou réglée en amplitude par un abaisseur (12) commandé par un micro-contrôleur (16). Selon un premier mode de réalisation, le micro-contrôleur (16) dialogue avec une interface entrée/sortie (18) commandant d'une part un panneau d'affichage et/ou assurant la liaison avec un micro-ordinateur. Selon un second mode de réalisation, le micro-contrôleur (16) dialogue un micro-ordinateur qui se trouve dans la console.

L'équipement comprend en outre un circuit (17) de détection et de traitement du signal provenant d'un implant.

Un circuit de temporisation interrompt l'alimentation de l'antenne après une durée cumulée d'émission prédéterminée pour éviter un échauffement excessif.

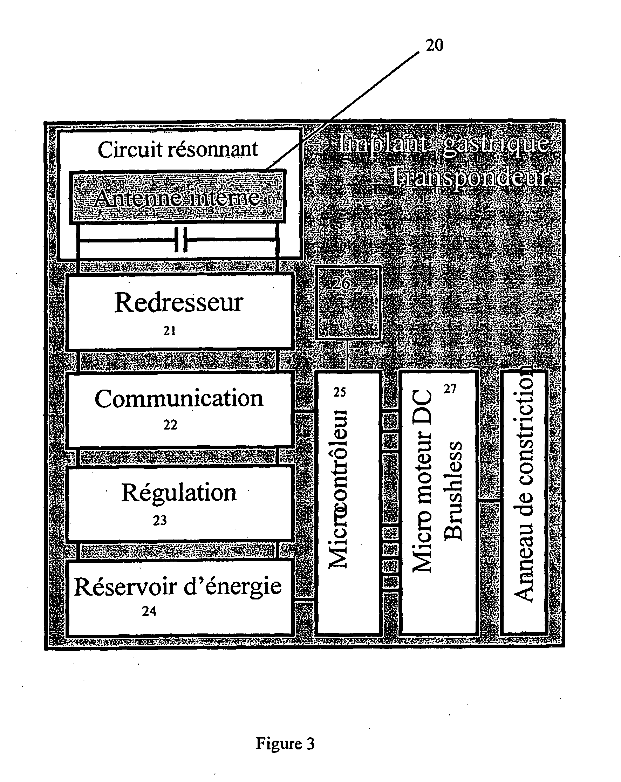

Le schéma de principe du circuit électronique de l'implant est représenté en figure 3. Il comprend une bobine d'induction (20) reliée par un redresseur (21) à un circuit de communication (22).

Selon un premier mode de réalisation de l'invention, une alimentation (24) sous forme d'une batterie tampon ou d'un condensateur, ou encore de tout moyen équivalent alimente les circuits électroniques par l'intermédiaire d'un circuit de régulation (23). Selon un second mode de réalisation, la bobine (20) alimente les circuits électroniques.

Un micro-contrôleur (25) commande le moteur sans balais (26), ainsi que les mémoires intégrées au micro-contrôleur (25).

Le circuit électronique de l'implant commande les fonctions suivantes :
- Génération d'une trame spécifique ou « trame de localisation » pour la localisation de l'équipement de commande lors du positionnement de l'antenne ;
- Gestion des mémoires, et notamment du verrouillage des informations enregistrées avant l'implantation par modification de l'état d'un drapeau empêchant de nouvelles écritures dans les registres correspondants ;
- Envoi de données consistant à la génération de messages, sous l'action d'une commande envoyée par l'équipement de commande ;
- Déplacement du manchon fileté, en fonction des consignes émises par l'équipement de commande. Le déplacement est commandé sans capteurs, avec un acquittement du déplacement par l'implant à chaque position avant toute nouvelle commande de déplacement ;
- Repositionnement de l'anneau : cette fonction est activée par l'équipement de commande, sous contrôle de l'opérateur lorsqu'il apparaît une incertitude sur la position effective du moteur par rapport aux informations enregistrées. L'équipement de commande envoie un message commandant le déplacement en butée du moteur de l'implant, en extension maximale et minimale, pour remettre à zéro l'information de position et calculer les positions limites.

Par ailleurs, le circuit de l'implant génère également des signaux d'alerte en cas de dysfonctionnement :
- Détection de blocage : ce signal est généré lorsqu'un signal de commande de déplacement est détecté, et que le moteur reste inactif ; ce blocage est détecté suivant un premier mode de réalisation parce qu'aucune modification du signal n'est fournie par le détecteur de position angulaire du moteur et suivant un second mode de réalisation en l'absence de capteur, en fonction du fait que le moteur ne démarre pas ou qu'il ralentit fortement. Le signal d'erreur est enregistré dans une mémoire de l'implant. Dans le premier mode de réalisation, le système génère une commande de retour. Dans le second mode de réalisation, l'implant retourne en position antécédente.

L'équipement de commande présente plusieurs états possibles :
- Arrêt : l'alimentation électrique est interrompue et les circuits électroniques sont au repos. L'antenne n'émet aucun rayonnement ;
- Attente : l'alimentation est activée et les circuits sont alimentés, l'antenne n'émet aucun rayonnement ;
- Utilisation normale : l'antenne est alimentée, l'implant est identifié ;
- Utilisation/localisation : l'antenne est alimentée, et l'équipement est en phase d'ajustement de la puissance d'émission par incrémentation périodique de la puissance et mesure de la réponse de l'implant, avec repositionnement de l'antenne pour optimiser la puissance transmise ;
- Utilisation/commande : l'énergie est fixée, et l'équipement transmet les informations de commande à l'implant ;
- Utilisation/alarme : l'implant ou l'équipement génère un signal d'alerte.

Un temporisateur active le mode d'attente après une utilisation d'une durée supérieure à une valeur prédéterminée.

De même, l'implant présente différents états de fonctionnement :
- Attente : il s'agit de l'état par défaut. Les circuits électroniques et le moteur ne sont pas alimentés ;
- Localisation : l'implant détecte une émission et dialogue avec un équipement en vue d'optimiser la puissance d'émission. Le but est de fixer la puissance d'émission à un niveau minimal requis pour transmettre une puissance suffisante au fonctionnement des circuits électroniques puis au moteur brushless, avec une détection sans équivoque des signaux de commande modulant en amplitude la tension appliquée à l'antenne de l'équipement de commande ;
- Prêt : l'implant est prêt à recevoir des signaux de commande ;
- Alarme : l'implant génère un signal d'alarme en réponse à une anomalie de fonctionnement ;
- Veille : l'implant ne répond plus, suite à une trop longue inactivité de l'équipement de commande.

La description qui suit concerne un mode de fonctionnement avantageux d'un système selon l'invention et précise quelques fonctionnalités importantes.

L'équipement de commande procède avant toute émission d'un signal de commande à l'identification de l'opérateur médical. A cet effet, il comporte une procédure de saisie d'un mot de passe et de vérification à l'aide d'un algorithme cryptologique par exemple. L'acquittement de cette procédure ouvre une session qui prend fin après une période d'inactivité déterminée.

La gestion des mots de passe et des utilisateurs, et notamment d'un nouvel utilisateur s'effectue à partir d'une procédure protégée par un accès maître.

La création d'un nouveau patient se fait à partir de l'ouverture d'une session d'un utilisateur référencé d'un plus haut niveau. L'équipement de commande comporte un clavier pour la saisie des informations spécifiques à un nouveau patient, telles qu'un identifiant, et pour l'enregistrement de ces informations dans la mémoire d'un nouvel implant. Ces données sont verrouillées lors de la première activation du moteur de l'implant. L'équipement de commande comprend à cet effet un algorithme neutralisant l'accès en écriture de la zone de mémoire destinée à recevoir les informations propres à l'identité du patient, en réponse à un signal par exemple après la première demande de déplacement du moteur.

Avant l'implantation, le système permet de réaliser une vérification du bon fonctionnement. A cet effet, l'équipement de commande transmet des instructions pour s'assurer de la rotation du moteur dans un sens puis dans l'autre, d'un seul pas.

La commande d'un implant comporte une étape de positionnement de l'antenne par le praticien. Il déplace l'antenne par rapport au corps du patient jusqu'à obtenir, pour une puissance donnée, le meilleur signal de réponse. La puissance est ensuite diminuée incrémentalement jusqu'à atteindre la puissance minimale compatible avec une alimentation satisfaisante de l'implant.

Après affichage des données mémorisées dans l'implant, le praticien peut alors commander le déplacement du moteur, soit par calcul d'un déplacement cible, soit par modification pas à pas du positionnement du moteur.

Dans le premier cas, l'équipement de commande recueille la position actuelle de l'implant par lecture de la mémoire de position de l'implant, puis calcule le nombre de positions nécessaires pour la position cible. L'équipement module alors le signal de commande à l'implant pour commander selon un protocole sécurisé le déplacement du moteur de l'implant.

Un autre mode d'utilisation consiste à envoyer signal de commande pour assurer le déplacement d'un pas, dans un sens ou dans l'autre.

Chaque déplacement se fait pas à pas pour s'assurer de bon déroulement des déplacements.

Ce mode permet d'augmenter ou de réduire la section de l'anneau gastrique.

Chaque déplacement effectué par l'implant est enregistré dans la mémoire de l'implant de façon à conserver l'historique des modifications. Seule la dernière modification est horodatée.

Le système comprend par ailleurs une procédure d'ouverture d'urgence permettant de commander l'ouverture maximale de l'anneau sans passer par une étape d'identification préalable.

Le fonctionnement de l'implant comprend différentes procédures. La procédure d'initialisation consiste à générer une trame spécifique pouvant être détectée par l'équipement de commande pendant la phase d'ajustement de la puissance appliquée à l'antenne.

Cette procédure est déclenchée par la détection par l'implant d'un signal modulé spécifique à l'équipement de commande, afin notamment de limiter le risque d'interaction avec d'autres appareillages à induction. Elle est interrompue, par exemple, lors de l'émission d'un signal d'acquittement par l'équipement de commande.

La procédure d'envoi de données est déclenchée par des signaux de commande spécifiques. Elle consiste à lire les informations enregistrées dans la mémoire de l'implant et à les transmettre à l'équipement de commande.

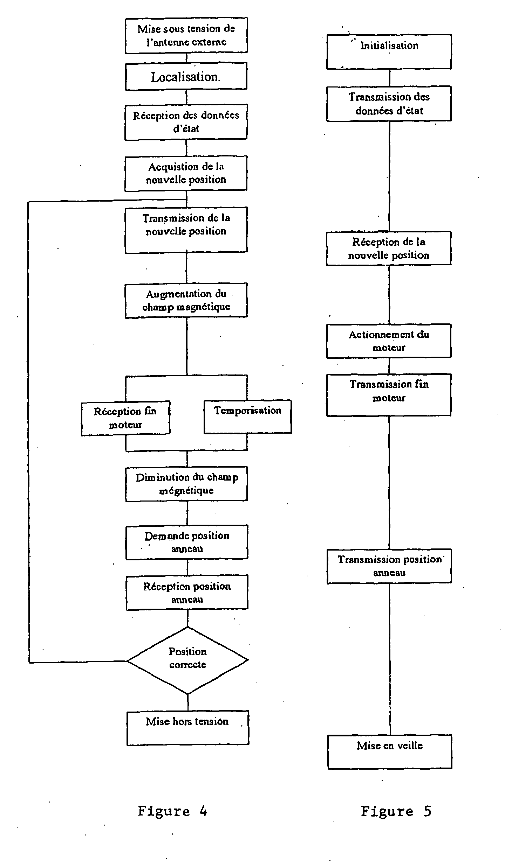

L'invention est décrite dans ce qui précède en référence à un exemple préféré de réalisation. Des exemples d'algorithmes de fonctionnement de l'équipement de commande et de l'implant sont illustrés respectivement par les figures 4 et 5.

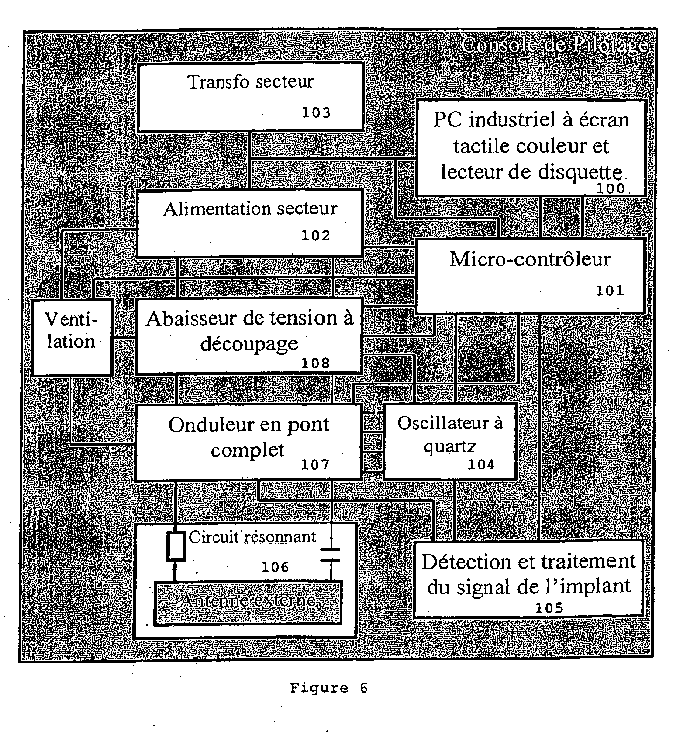

La figure 6 représente un schéma de principe d'une variante de réalisation de l'équipement de commande (console de pilotage - émetteur / récepteur).

La console de pilotage de cette variante de réalisation est constitué par une carte de commande et un ordinateur industriel (100) comportant un écran tactile.

La dalle tactile permet de créer des menus contextuels par un logiciel applicatif chargé dans la mémoire vive de l'ordinateur. Ce logiciel applicatif active les zones tactiles correspondant seulement aux fonctions autorisées compte tenu de l'état du système et inhibant toutes les autres actions. Cette solution permet d'augmenter la sécurité d'utilisation du système.

Certaines fonctions de sécurités telles que la gestion des mots de passe sont assurée non pas par l'ordinateur (100), mais par le micro-contrôleur (101) de la carte de commande de l'anneau gastrique.

L'ordinateur assure les fonctions d'interface homme-machine, les fonctions de commande de l'implant restant assurées par les circuits électroniques de la carte de commande. Un périphérique, par exemple un lecteur de disquettes, permet de sauvegarder et/ou de traiter les données mémorisées.

Cette carte de commande comprend, outre le micro-contrôleur (101), une alimentation (102) comprenant un transformateur secteur (103), un oscillateur à quartz (104) délivrant un signal d'horloge et un circuit (105) de détection et de traitement des signaux échangés avec l'implant.

L'alimentation et la communication avec l'implant est assurée par une antenne (106) alimentée par un onduleur en pont complet (107). Selon cette variante, la tension d'alimentation de l'antenne (106) est abaissée par un convertisseur de tension (108) à découpage. Ceci permet de limiter la puissance d'émission au strict nécessaire pour le bon fonctionnement de l'implant. L'antenne de l'implant est formée par un cadre plastique autour duquel est bobiné un bobinage, par exemple des spires de fil de cuivre émaillé. Le cadre est intégré dans l'implant, ce qui permet de fiabiliser la construction de l'implant. Suivant un premier mode de réalisation, on utilise une antenne mince à l'extérieur du boîtier qui doit être insérée dans le corps du patient sous une forme enroulée. Suivant un second mode de réalisation, l'antenne est placée dans le boîtier : on évite ainsi les risques de panne par rupture de l'antenne mince après insertion.

La fréquence de communication est fixée de 115,2 Khz à 120,6 Khz pour permettre une transmission optimale de puissance à une distance inférieure à 10 centimètres.

## Revendications

1. Système pour le traitement de l'obésité comprenant un implant gastrique pour le contrôle de l'absorption alimentaire et un équipement de commande, l'implant gastrique comportant un anneau apte à enserrer l'oesophage et un moyen de commande motorisé pour l'ajustement de l'extension dudit anneau, ainsi qu'une bobine d'induction (20) pour l'alimentation sans contact du moyen de commande motorisé **caractérisé en ce que** la bobine d'induction (20) de l'implant est intégrée à l'implant et **en ce que** l'équipement de commande comporte des moyens pour l'ajustement de la puissance d'émission et **en ce que** l'implant comporte un circuit électronique (8) délivrant un signal de réponse à une commande transmise par l'équipement de commande.

2. Système selon la revendication 1, **caractérisé en ce que** le circuit électronique (8) de l'implant comporte une mémoire pour l'enregistrement de données numériques d'identification de l'implant.

3. Système selon la revendication 1, **caractérisé en ce que** le circuit électronique (8) de l'implant comporte une mémoire pour l'enregistrement de données numériques d'identification du patient.

4. Système selon la revendication 1, **caractérisé en ce que** le circuit électronique (8) de l'implant comporte une mémoire pour l'enregistrement de données numériques correspondant à l'historique.

5. Système selon l'une quelconque des revendications précédentes, **caractérisé en ce que** l'implant comporte une mémoire pour l'enregistrement de la position absolue de l'anneau.

6. Système selon l'une quelconque des revendications précédentes, **caractérisé en ce que** l'implant comporte une mémoire pour l'enregistrement de limites de déplacement de l'anneau, et un moyen pour empêcher la commande du moteur en cas de réception de commandes conduisant à un dépassement desdites limites.

7. Système selon l'une quelconque des revendications précédentes, **caractérisé en ce que** l'équipement de commande comprend des moyens de réception des informations enregistrées dans la mémoire d'un implant et une mémoire pour l'enregistrement de l'historique, comprenant l'identifiant de chacun des implants commandés et la date de modification de chacun desdits implants.

8. Système selon l'une quelconque des revendications précédentes, **caractérisé en ce que** l'équipement de commande comprend une antenne mobile (13, 106) et un moyen de génération d'une trame numérique de référence pour l'étape d'ajustement de la puissance d'émission préalable à l'étape de commande d'un anneau implanté.

9. Système selon l'une quelconque des revendications précédentes, **caractérisé en ce que** l'équipement de commande comprend un circuit de réglage en amplitude de la tension d'alimentation de l'antenne mobile (13, 106).

10. Système selon l'une quelconque des revendications précédentes, **caractérisé en ce que** l'implant comporte un circuit de temporisation activant le mode veille après une période prédéterminée sans signaux de commande.

11. Système selon l'une quelconque des revendications précédentes, **caractérisé en ce qu'**il comprend une fonction de test commandant la fermeture d'une position puis l'ouverture d'une position de l'implant, l'équipement de commande comportant une information de désactivation de la fonction de test.

12. Système selon l'une quelconque des revendications précédentes, **caractérisé en ce que** l'équipement de commande comporte un calculateur pour la fabrication d'un signal de commande qui est fonction d'une position saisie par un opérateur et les informations de contrôle provenant de l'implant à commander.

13. Système selon l'une quelconque des revendications précédentes, **caractérisé en ce que** l'équipement de commande est apte à générer un signal sécurisé pour le déplacement d'un pas du moteur de l'implant.

14. Système selon l'une quelconque des revendications précédentes, **caractérisé en ce que** la bobine d'induction (20) de l'implant est constituée par un cadre comportant un bobinage ledit cadre étant intégré à l'implant.

15. Système selon l'une quelconque des revendications précédentes, **caractérisé en ce que** le circuit de pilotage comprend un convertisseur de tension (108) à découpage inséré entre l'alimentation (24, 102) et l'onduleur en pont complet (14, 107) permettant de limiter la puissance d'émission.

16. Implant gastrique pour un système conforme à l'une au moins des revendications précédentes, **caractérisé en ce qu'**il comprend un anneau apte à enserrer l'oesophage et un moyen de commande motorisé pour l'ajustement de l'extension dudit anneau, ainsi qu'une bobine d'induction (20) pour l'alimentation sans contact du moyen de commande motorisé intégrée à l'implant et **en ce qu'**il comporte un circuit électronique (8) délivrant un signal de réponse à une commande transmise par l'équipement de commande.

17. Implant gastrique selon la revendication 16 **caractérisé en ce que** l'alimentation (24, 102) est réalisée par un bobinage formé autour d'un cadre, ledit cadre étant intégré à l'implant.

## Patentansprüche

1. System zur Behandlung von Fettsucht, das ein Magenimplantat zur Kontrolle der Nahrungsmittelabsorption und eine Steuerausrüstung enthält, wobei das Magenimplantat einen Ring, der die Speiseröhre umspannen kann, und eine motorisierte Steuereinrichtung zur Anpassung der Ausdehnung des Rings sowie eine Induktionsspule (20) zur berührungslosen Speisung der motorisierten Steuereinrichtung aufweist, **dadurch gekennzeichnet, dass** die Induktionsspule (20) des Implantats in das Implantat integriert ist, und dass die Steuerausrüstung Einrichtungen zur Anpassung der Sendeleistung aufweist, und dass das Implantat eine elektronische Schaltung (8) aufweist, die ein Antwortsignal auf einen von der Steuerausrüstung übertragenen Befehl liefert.

2. System nach Anspruch 1, **dadurch gekennzeichnet, dass** die elektronische Schaltung (8) des Implantats einen Speicher zum Speichern von digitalen Identifikationsdaten des Implantats aufweist.

3. System nach Anspruch 1, **dadurch gekennzeichnet, dass** die elektronische Schaltung (8) des Implantats einen Speicher zum Speichern von digitalen Identifikationsdaten des Patienten aufweist.

4. System nach Anspruch 1, **dadurch gekennzeichnet, dass** die elektronische Schaltung (8) des Implantats einen Speicher zum Speichern von dem Verlauf entsprechenden digitalen Daten aufweist.

5. System nach einem der vorhergehenden Ansprüche, **dadurch gekennzeichnet, dass** das Implantat einen Speicher zum Speichern der Absolutposition des Rings aufweist.

6. System nach einem der vorhergehenden Ansprüche, **dadurch gekennzeichnet, dass** das Implantat einen Speicher zum Speichern von Verschiebegrenzen des Rings und eine Einrichtung aufweist, um die Steuerung des Motors im Fall des Empfangs von Befehlen zu verhindern, die zu einem Überschreiten der Grenzen führen.

7. System nach einem der vorhergehenden Ansprüche, **dadurch gekennzeichnet, dass** die Steuerausrüstung Empfangseinrichtungen der im Speicher eines Implantats gespeicherten Informationen und einen Speicher zum Speichern des Verlaufs enthält, der die Kennung jedes der gesteuerten Implantate und das Änderungsdatum jedes der Implantate enthält.

8. System nach einem der vorhergehenden Ansprüche, **dadurch gekennzeichnet, dass** die Steuerausrüstung eine bewegliche Antenne (13, 106) und eine Einrichtung zur Erzeugung eines digitalen Bezugsrahmens für den Schritt der Anpassung der Sendeleistung vor dem Schritt der Steuerung eines implantierten Rings aufweist.

9. System nach einem der vorhergehenden Ansprüche, **dadurch gekennzeichnet, dass** die Steuerausrüstung eine Amplitudenregelungsschaltung der Speisespannung der beweglichen Antenne (13, 106) enthält.

10. System nach einem der vorhergehenden Ansprüche, **dadurch gekennzeichnet, dass** das Implantat eine Verzögerungsschaltung aufweist, die den Standby-Modus nach einer vorbestimmten Zeitspanne ohne Steuersignale aktiviert.

11. System nach einem der vorhergehenden Ansprüche, **dadurch gekennzeichnet, dass** es eine Testfunktion enthält, die das Schließen einer Position und dann das Öffnen einer Position des Implantats steuert, wobei die Steuerausrüstung eine Deaktivierungsinformation der Testfunktion aufweist.

12. System nach einem der vorhergehenden Ansprüche, **dadurch gekennzeichnet, dass** die Steuerausrüstung einen Rechner zur Herstellung eines Steuersignals, das von einer von einem Bediener eingegebenen Position abhängt, und die vom zu steuernden Implantat stammenden Kontrollinformationen aufweist.

13. System nach einem der vorhergehenden Ansprüche, **dadurch gekennzeichnet, dass** die Steuerausrüstung ein gesichertes Signal für die Verschiebung des Motors des Implantats um einen Schritt erzeugen kann.

14. System nach einem der vorhergehenden Ansprüche, **dadurch gekennzeichnet, dass** die Induktionsspule (20) des Implantats aus einem Rahmen besteht, der eine Wicklung aufweist, wobei der Rahmen in das Implantat integriert ist.

15. System nach einem der vorhergehenden Ansprüche, **dadurch gekennzeichnet, dass** die Steuerschaltung einen Spannungswandler (108) mit Zerhackung aufweist, der zwischen die Speisung (24, 102) und den Vollbrücken-Wechselrichter (14, 107) eingefügt ist, der die Begrenzung der Sendeleistung ermöglicht.

16. Magenimplantat für ein System nach mindestens einem der vorhergehenden Ansprüche, **dadurch gekennzeichnet, dass** es einen Ring, der die Speiseröhre umspannen kann, und eine motorisierte Steuereinrichtung zur Anpassung der Ausdehnung des Rings sowie eine Induktionsspule (20) für die berührungslose Speisung der motorisierten Steuereinrichtung enthält, die in das Implantat integriert ist, und dass es eine elektronische Schaltung (8) aufweist, die ein Antwortsignal auf einen von der Steuerausrüstung übertragenen Befehl liefert.

17. Magenimplantat nach Anspruch 16, **dadurch gekennzeichnet, dass** die Speisung (24, 102) von einer Wicklung ausgeführt wird, die um einen Rahmen herum geformt ist, wobei der Rahmen in das Implantat integriert ist.

## Claims

1. System for treating obesity, comprising a gastric implant for controlling food absorption, and control equipment, the gastric implant having a ring for enclosing the oesophagus and a motorized controller for adjusting the extension of said ring, and also an induction coil (20) for the contactless powering of the motorized controller, **characterized in that** the induction coil (20) of the implant is integrated in the implant, and **in that** the control equipment has means for adjusting the transmission power, and **in that** the implant has an electronic circuit (8) delivering a response signal to a command transmitted by the control equipment.

2. System according to Claim 1, **characterized in that** the electronic circuit (8) of the implant has a memory for recording digital data identifying the implant.

3. System according to Claim 1, **characterized in that** the electronic circuit (8) of the implant has a memory for recording digital data identifying the patient.

4. System according to Claim 1, **characterized in that** the electronic circuit (8) of the implant has a memory for recording digital data corresponding to historical information.

5. System according to any one of the preceding claims, **characterized in that** the implant has a memory for recording the absolute position of the ring.

6. System according to any one of the preceding claims, **characterized in that** the implant has a memory for recording movement limits of the ring, and a means for preventing control of the motor in the event of commands being received that would lead to said limits being exceeded.

7. System according to any one of the preceding claims, **characterized in that** the control equipment comprises means for receiving the information recorded in the memory of an implant and a memory for recording the historical information, including the identifier of each of the controlled implants and the date of modification of each of said implants.

8. System according to any one of the preceding claims, **characterized in that** the control equipment comprises a mobile antenna (13, 106) and a means for generating a digital reference screen for the step of adjusting the transmission power prior to the step of controlling an implanted ring.

9. System according to any one of the preceding claims, **characterized in that** the control equipment comprises a circuit for regulating the amplitude of the supply voltage of the mobile antenna (13, 106).

10. System according to any one of the preceding claims, **characterized in that** the implant has a timeout circuit for activating the standby mode after a predetermined period without control signals.

11. System according to any one of the preceding claims, **characterized in that** it comprises a test function controlling the closure of a position and then the opening of a position of the implant, the control equipment having information for deactivating the test function.

12. System according to any one of the preceding claims, **characterized in that** the control equipment comprises a calculator for creating a control signal which is a function of a position input by an operator and command information originating from the implant to be controlled.

13. System according to any one of the preceding claims, **characterized in that** the control equipment is able to generate a secure signal for displacement, by one step, of the motor of the implant.

14. System according to any one of the preceding claims, **characterized in that** the induction coil (20) of the implant is composed of a frame that comprises a winding, said frame being integrated in the implant.

15. System according to any one of the preceding claims, **characterized in that** the command circuit comprises a cut-off voltage converter (108) inserted between the power supply (24, 102) and the full-bridge inverter (14, 107) making it possible to limit the transmission power.

16. Gastric implant for a system according to at least one of the preceding claims, **characterized in that** it comprises a ring for enclosing the oesophagus, and a motorized controller for adjusting the extension of said ring, and also an induction coil (20) for the contactless powering of the motorized controller integrated in the implant, and **in that** it has an electronic circuit (8) delivering a response signal to a command transmitted by the control equipment.

17. Gastric implant according to Claim 16, **characterized in that** the power supply (24, 102) is provided by a winding formed around a frame, said frame being integrated in the implant.
